# EUROPEAN PATENT APPLICATION

(11) **EP 2 022 782 A1**
(43) Date of publication of application: **11.02.2009**
(21) Application number: 07744543.5
(22) Date of filing: 01.06.2007
(51) Int. Cl.: C07D 211/46, C07D 211/58, C07D 211/62, C07D 401/12, C07D 405/12, A61K 31/451, A61K 31/453, A61K 31/454, A61K 31/4545, A61K 31/5377, A61K 31/541, A61P 1/00, A61P 1/08, A61P 9/00, A61P 13/02, A61P 25/00, A61P 25/04, A61P 25/24, A61P 29/00, A61P 37/08

(54) **PROCESS FOR PRODUCTION OF OPTICALLY ACTIVE PIPERIDINE COMPOUND**

(30) Priority: 01.06.2006 JP 2006153605
(71) Applicant: Mitsubishi Tanabe Pharma Corporation, Chuo-ku Osaka-shi Osaka 541-8505 (JP)
(72) Inventor: MATSUMAE, Hiroaki, 3-chome, Chuo-ku, Osaka-shi,Osaka 541-8505 (JP); MORI, Yoshikazu, 3-chome, Chuo-ku, Osaka-shi,Osaka 541-8505 (JP); MATSUYAMA, Koji, 3-chome, Chuo-ku, Osaka-shi,Osaka 541-8505 (JP); MORIYAMA, Noriaki, 3-chome, Chuo-ku, Osaka-shi,Osaka 541-8505 (JP)
(74) Representative: HOFFMANN EITLE
(86) International application number: PCT/JP2007/061155
(87) International publication number: WO 2007/139211

(57) **Abstract**

The present invention relates to a method for preparing a syn-form piperidine compound represented by general formula [I]: 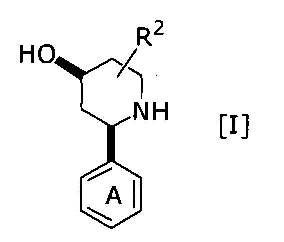 wherein,
bold lines represent bonds in which substituents at positions 2 and 4 of a piperidine ring are in the syn configuration, and the other symbols have the same meaning as defined below, or a salt thereof,
comprising:
reducing a compound represented by general formula [II]:

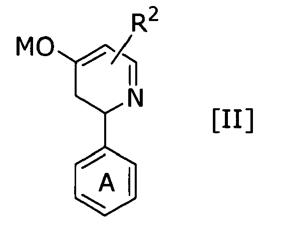

wherein,
ring A represents an optionally substituted benzene ring,
R² represents a hydrogen atom, an optionally substituted hydroxyl group, an optionally substituted amino group, an optionally substituted alkyl group, a substituted carbonyl group or a halogen atom, and
M represents an alkaline metal or hydrogen atom.

## Description

### TECHNICAL FIELD

The present invention relates to a method for preparing intermediates of piperidine compounds having excellent tachykinin receptor antagonistic activity, and to a method for preparing piperidine compounds having excellent tachykinin receptor antagonistic activity using those intermediates.

### BACKGROUND ART

Tachykinin is the generic term for a group of neuropeptides, and are known as substance P (hereinafter referred to as SP), neurokinin A and neurokinin B in mammals. These peptides are known to demonstrate various biological activities by binding to their respective receptors present in the living body (neurokinin-1, neurokinin-2 and/or neurokinin-3). Among these, SP is one of the neuropeptides that have the longest history and have been studied in detail among neuropeptides. SP has been confirmed in 1931 to be present in equine intestinal tract extracts, and is a peptide comprising 11 amino acids, whose structure was determined in 1971.

SP is widely distributed in the central and peripheral nervous systems, and in addition to functioning as a primary sensory neurotransmitter, SP has physiological activity such as vasodilatory action, vascular permeability enhancing action, smooth muscle-contracting action, neuron excitatory action, salivary secretion action, diuresis-enhancing action and immunologic action. In particular, it is known that SP released from terminals of the spinal dorsal horn in response to a pain impulse transmits pain information to secondary neurons, and that SP released from peripheral terminals causes an inflammatory reaction in receptors thereof. On the basis of these findings, SP is considered to be involved in various pathological states (e.g., pain, inflammation, allergies, pollakiuria, urinary incontinence, respiratory tract diseases, psychoses, depression, anxiety, vomiting, etc). SP is also considered to be involved in Alzheimer's dementia [Introduction: Physiological Reviews, Vol. 73, pp. 229-308 (1993) (Non-Patent Document 1) and Journal of Autonomic Pharmacology, Vol. 13, pp. 23-93 (1993) (Non-Patent Document 2)].

As an example of a compound having tachykinin receptor antagonistic activity, Patent Document 1 discloses a piperidine derivative represented by the following formula: 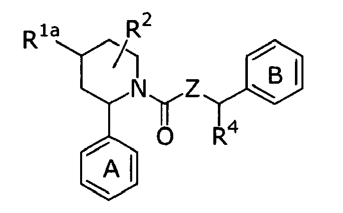 wherein,
ring A represents an optionally substituted benzene ring, ring B represents an optionally substituted benzene ring, R^{1a} represents an optionally substituted alkyl group, an optionally substituted hydroxyl group, a substituted thiol group, a substituted carbonyl group, a substituted sulfinyl group, a substituted sulfonyl group, or a group represented by the formula:

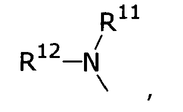

R¹¹ and R¹² may be the same or different and each represents a hydrogen atom, a substituted carbonyl group, a substituted sulfonyl group, an optionally substituted alkyl group or a heterocyclic group containing as a heteroatom 1 to 4 atoms selected from nitrogen atom, oxygen atom and sulfur atom, wherein the heterocyclic group is optionally substituted and the nitrogen atom contained in the heterocyclic group is optionally oxidized, or R¹¹ and R¹², together with the nitrogen atom to which they are attached, form a heterocyclic group selected from a piperidino group, azacycloheptyl group, pyrrolidino group, imidazolidinyl group, hexahydropyrimidinyl group, thiazolidyl group, morpholino group, triazolyl group, tetrazolyl group and purinyl group, wherein the heterocyclic group is optionally substituted, and the nitrogen atom contained in the heterocyclic group is optionally oxidized, R² represents a hydrogen atom, an optionally substituted hydroxyl group, an optionally substituted amino group, an optionally substituted alkyl group, a substituted carbonyl group or a halogen atom, Z represents an oxygen atom or a group represented by - N(R³)-, wherein R³ represents a hydrogen atom or an optionally substituted alkyl group, and R⁴ represents a hydrogen atom or an optionally substituted alkyl group, or a pharmaceutically acceptable salt thereof, and also discloses a method for preparing their intermediates.
[Patent Document 1] International Publication No. WO 2003/099787
[Non-Patent Document 1] Physiological Reviews, Vol. 73, pp. 229-308 (1993)
[Non-Patent Document 2] Journal of Anatomic Pharmacology, Vol. 13, pp. 23-93 (1993)

### DISCLOSURE OF THE INVENTION

### Problems to be Solved by the Invention

Optically active compounds which are intermediates of tachykinin receptor antagonists were unable to be efficiently prepared using known preparation methods, and these methods had problems in terms of preparation costs. An object of the present invention is to provide an industrially advantageous method for preparing syn-form piperidine compounds, which are intermediates of tachykinin receptor antagonists, and optically active piperidine compounds, and to provide an industrially advantageous method for preparing optically active piperidine compounds having tachykinin receptor antagonistic action using these optically active intermediates.

### Means for Solving the Problems

The present invention relates to a method for preparing a syn-form piperidine compound represented by general formula [I]: 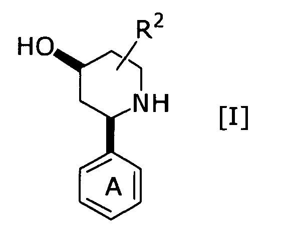 wherein,
bold lines represent bonds in which substituents at positions 2 and 4 of a piperidine ring are in the syn configuration, and the other symbols have the same meaning as defined below, or a salt thereof, comprising:
reducing a compound represented by general formula [II]: 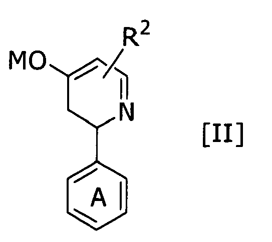
wherein,
ring A represents an optionally substituted benzene ring,
R² represents a hydrogen atom, an optionally substituted hydroxyl group, an optionally substituted amino group, an optionally substituted alkyl group, a substituted carbonyl group or a halogen atom, and
M represents an alkaline metal or a hydrogen atom.

The present invention also relates to a method for preparing a syn-form piperidine compound represented by general formula [I]: 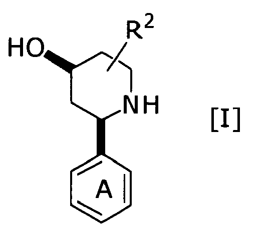 wherein,
bold lines represent bonds in which substituents at positions 2 and 4 of a piperidine ring are in the syn configuration, and the other symbols have the same meaning as defined below, or a salt thereof, comprising:
reacting a compound represented by general formula [III]: 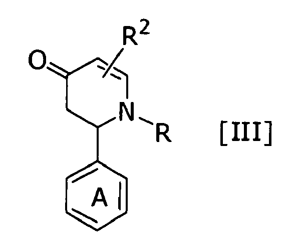
wherein,
ring A represents an optionally substituted benzene ring,
R² represents a hydrogen atom, an optionally substituted hydroxyl group, an optionally substituted amino group, an optionally substituted alkyl group, a substituted carbonyl group or a halogen atom, and
R represents a protecting group for an amino group, or a salt thereof,
with a base to give a compound represented by general formula [II]: 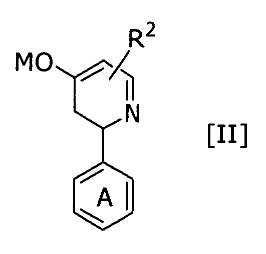 wherein,
M represents an alkaline metal or a hydrogen atom, and the other symbols have the same meaning as defined above, followed by
reducing the compound [II] obtained.

In a preferred embodiment of the aforementioned preparation method, ring A is a benzene ring represented by the formula: 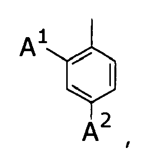 A¹ is a hydrogen atom, an alkyl group or a halogen atom, A² is a hydrogen atom, an alkyl group or a halogen atom, R² is a hydrogen atom, and R is an alkoxycarbonyl group or an arylalkoxycarbonyl group.

In a more preferred embodiment of the aforementioned preparation method, the base is an alkaline metal alcolate and M is an alkaline metal.

Moreover, the present invention relates to a method for preparing an optically active piperidine compound represented by general formula [I-a] or general formula [I-b] : 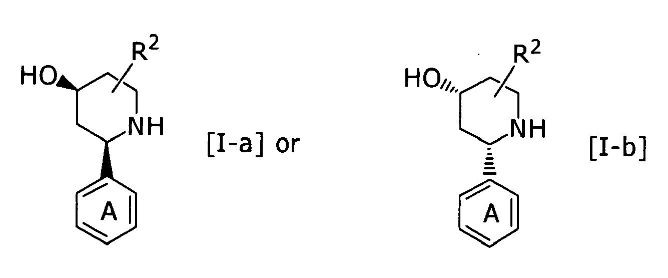 wherein,
the symbols have the same meaning as defined below, comprising:
preparing a syn-form piperidine compound represented by general formula [I]: 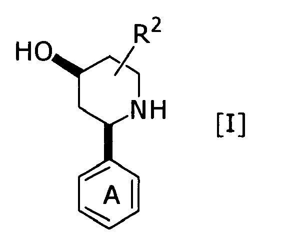
wherein,
ring A represents an optionally substituted benzene ring,
R² represents a hydrogen atom, an optionally substituted hydroxyl group, an optionally substituted amino group, an optionally substituted alkyl group, a substituted carbonyl group or a halogen atom, and
bold lines represent bonds in which substituents at positions 2 and 4 of a piperidine ring are in the syn configuration,
according to the method described above,
allowing the syn-form piperidine compound [I] obtained to act on an optical resolving agent to form two types of diastereomer salts,
using a difference in solubility between the two types of diastereomer salts formed to separate and collect one of the diastereomer salts, and then
decomposing said salt.

Moreover, the present invention relates to a method for preparing an optically active piperidine compound represented by general formula [V-a] or general formula [V-b] : 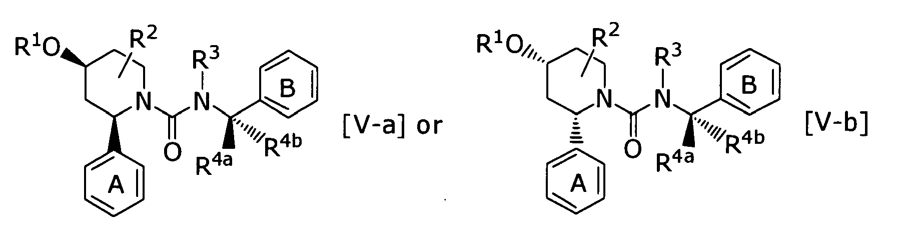 wherein,
the symbols have the same meaning as defined below, or a pharmaceutically acceptable salt thereof,
comprising:
optionally introducing a substituent R¹ to a hydroxyl group at position 4 of a piperidine ring of the optically active piperidine compound represented by general formula [I-a] or general formula [I-b] obtained according to the above method: 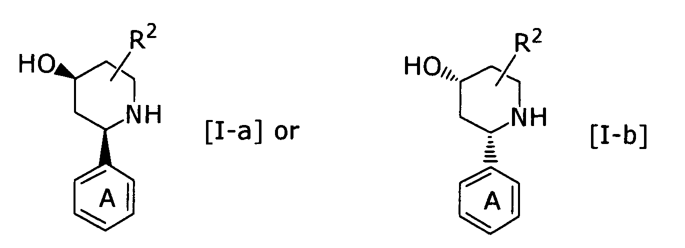
wherein,
the symbols have the same meaning as defined above, in accordance with usual manners to give a compound represented by general formula [I-c] or general formula [I-d] : 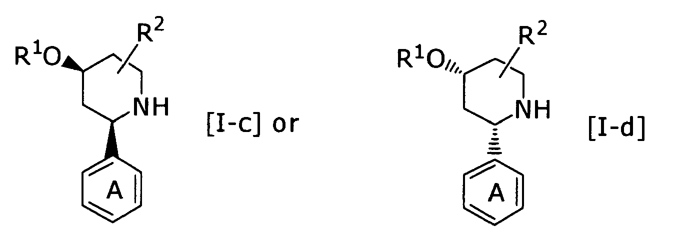 wherein,
R¹ represents a hydrogen atom, a substituted carbonyl group, a substituted sulfinyl group, a substituted sulfonyl group or an optionally substituted alkyl group, and the other symbols have the same meaning as defined above,
reacting the compound represented by general formula [I-c] or general formula [I-d], a compound represented by general formula [IV]: 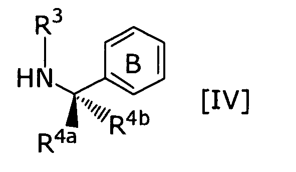 wherein,
ring B represents an optionally substituted benzene ring, R³ represents a hydrogen atom or an optionally substituted alkyl group, R^{4a} and R^{4b} may be the same or different and each represents a hydrogen atom or an optically substituted alkyl group, or R^{4a} and R^{4b} taken together form an alkylene group,
and an ureation agent represented by the formula: 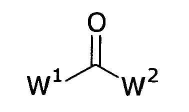 wherein,
W¹ and W² may be the same or different and each represents a leaving group, and then
as desired, converting to a pharmaceutically acceptable salt thereof.

### Effects of the Invention

The present invention provides a method for preparing syn-form piperidine compounds, which are intermediates of compounds having excellent tachykinin receptor antagonistic activity, and optically active piperidine compounds, and a method for preparing optically active piperidine compounds having excellent tachykinin receptor antagonistic activity using these optically active intermediates.

### BEST MODE FOR CARRYING OUT THE INVENTION

The compound [I] used or obtained in the present invention can be prepared by reducing the compound [II].

The reduction reaction of the compound [II] can be carried out in the presence of a reducing agent and in the presence or absence of a suitable solvent. The solvent may be any solvent which does not have a negative impact on the reaction, and examples thereof include alcohols such as methanol, ethanol, propanol, isopropanol, butanol, isobutanol, sec-butanol, tert-butanol and tetrahydrofurfuryl alcohol, ethers such as dimethoxyethane and tetrahydrofuran, aromatic hydrocarbons such as benzene and toluene, amides such as dimethylformamide and dimethylacetoamide, sulfoxides such as dimethylsulfoxide, nitriles such as acetonitrile, glycols such as ethylene glycol, water or a combination thereof. Alcohols are preferably used, and methanol or isopropanol is more preferably used. Examples of reducing agents that can be used include boron compounds such as sodium borohydride, lithium borohydride, calcium borohydride and zinc borohydride. Among them, sodium borohydride is preferably used. The amount of the reducing agent is preferably 1 to 3 equivalents and more preferably 1.8 to 2.2 equivalents based on the compound [II]. The reaction temperature of the reduction reaction is preferably 20 to 60°C and more preferably 30 to 50°C. The reaction time may vary according to the reaction temperature and the like, and is normally 30 minutes to 24 hours and preferably 1 to 20 hours. In the reduction reaction, the syn selectivity of substituents at positions 2 and 4 of the piperidine ring is extremely high at a syn/anti ratio of 9/1 or more, thereby enabling the syn-form compound [I] to be prepared stereoselectively and efficiently without using a special stereoselective reducing agent.

In addition, examples of M used in the present invention include alkaline metals such as lithium, potassium and sodium, or a hydrogen atom. Among them, sodium is preferably used.

The compound [I] used or obtained in the present invention can also be prepared by reacting the compound [III] or a salt thereof with a base to give the compound [II] followed by reducing the compound [II] obtained.

The reaction of the compound [III], or a salt thereof, with a base can be carried out in a suitable solvent. The solvent may be any solvent which does not have a negative impact on the reaction, and examples thereof include alcohols such as methanol, ethanol, propanol, isopropanol, butanol, isobutanol, sec-butanol, tert-butanol and tetrahydrofurfuryl alcohol, water or a combination thereof. Methanol or isopropanol is preferably used. The reaction temperature of this reaction is preferably 15 to 60°C and more preferably 45 to 55°C. The reaction time may vary according to the reaction temperature and the like, and is normally 30 minutes to 24 hours and preferably 1 to 20 hours.

Examples of bases used in the present invention include alkaline metal alcolates, alkaline metal hydroxides, alkaline earth metal hydroxides or the like. Among them, alkaline metal alcolates are preferably used. Examples of these alkaline metal alcolates include lithium methylate, lithium ethylate, lithium propylate, lithium isopropylate, potassium methylate, potassium ethylate, potassium propylate, potassium isopropylate, sodium methylate, sodium ethylate, sodium propylate, sodium isopropylate, lithium-tert-butoxide, potassium-tert-butoxide, sodium tert-butoxide and the like. Among them, sodium methylate is preferably used. In addition, examples of the alkaline metal hydroxides include lithium hydroxide, potassium hydroxide, sodium hydroxide and the like. Examples of the alkaline earth metal hydroxides include calcium hydroxide, magnesium hydroxide and the like.

The amount of a base is preferably 1 to 3 equivalents and more preferably 1.1 to 1.3 equivalents based on the compound [III].

The alkaline metal alcolate used in the present invention can be prepared within or outside the reaction system using an alkaline metal or alkaline metal salt and an alcohol. Examples of the alkaline metal include lithium, potassium, sodium and the like. Examples of the alkaline metal salt include alkaline metal hydroxides such as lithium hydroxide, potassium hydroxide and sodium hydroxide. Among them, sodium hydroxide is preferably used. In addition, examples of the alcohol include methanol, ethanol, propanol, butanol and the like. Among them, methanol is preferably used. The amount of the alkaline metal alcolate is preferably 1 to 3 equivalents and more preferably 1.1 to 1.3 equivalents based on the compound [III].

In a preferred embodiment of the present invention, the base is an alkaline metal alcolate and M is an alkaline metal. In a more preferred embodiment of the present invention, the base is sodium methylate and M is sodium.

The compound [I] of the present invention can be isolated and purified in accordance with usual manners.

A known example of a preparation method of the compound [V] is described in International Publication No. WO 2003/099787, but the yield is not necessarily good. In the present invention, the compound [V] can be prepared efficiently, and can be prepared, for example, in the manner described below. 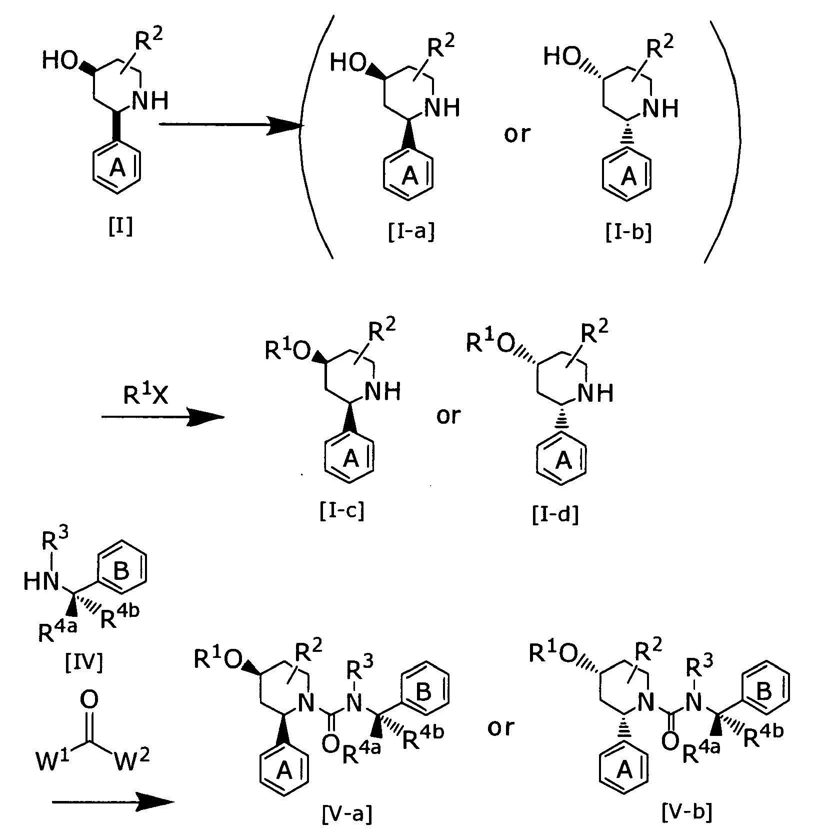 (wherein, W¹ and W² may be the same or different and each represents a leaving group, X represents a leaving group, and the other symbols have the same meaning as defined above).

The compound [I-a] or the compound [I-b] can be obtained by optical resolution in accordance with usual manners. Optical resolution can be carried out by, for example, allowing the compound [I] to act on an optical resolving agent to form two types of diastereomer salts, and then using a difference in solubility between the two types of diastereomer salts formed to separate and collect one of the diastereomer salts.

Examples of optical resolving agents that are preferably used include, for example, naproxen, N-o-dibenzoyl-D-p-hydroxyphenylglycine, di-p-anisoyl-D-tartaric acid, N-tosyl-L-phenylglycine, di-p-toloyl-L-tartaric acid, N-tosyl-D-valine, di-o-toloyl-D-tartaric acid, N-tosyl-D-phenylalanine, N-benzoyl-D-phenylglycine, L-methoxyacetic acid, N-benzenesulfonyl-D-phenylalanine and the like. In particular, in the case of obtaining the compound [I-a] in (2R,4S) form , N-tosyl-D-phenylalanine is preferably used. The amount of the optical resolving agent is preferably 0.5 to 0.8 equivalents and more preferably 0.6 to 0.7 equivalents based on the compound [I].

The reaction of the compound [I] with the optical resolving agent can be carried out in a suitable solvent. The solvent may be any solvent which does not have a negative impact on the reaction. Examples thereof include alcohols such as methanol and ethanol. Among them, methanol is preferably used. The reaction temperature of the reaction of the compound [I] with the optical resolving agent is preferably 5 to 70°C and more preferably 55 to 65°C. The reaction time may vary according to the reaction temperature and the like, and is normally 30 minutes to 24 hours and preferably 1 to 20 hours.

The two types of diastereomer salts formed by this reaction can be separated and collected by using usual manners such as fractional crystallization using the difference in solubility between the two diastereomer salts.

The reaction for decomposing the one diastereomer salt obtained to a free form can be carried out by reacting the salt with an acid or base in accordance with usual manners. This reaction can be carried out in a suitable solvent. The solvent may be any solvent which does not have a negative impact on the reaction, and examples thereof include water, esters such as ethyl acetate, isopropyl acetate and butyl acetate, halogenated hydrocarbons such as chloroform and dichloromethane, aromatic hydrocarbons such as benzene and toluene, or a combination thereof. Among them, a combination of water and isopropyl acetate is preferably used. Examples of the acid or base used include commonly used acids or bases including inorganic acids such as hydrochloric acid and sulfuric acid or organic amines such as pyridine and triethylamine, and hydrochloric acid is preferably used. The reaction temperature of this reaction is preferably 30 to 60°C and more preferably 35 to 45°C. The reaction time may vary according to the reaction temperature and the like, and is normally 30 minutes to 24 hours and preferably 1 to 20 hours.

The compound [I-c] or the compound [I-d] can be prepared in accordance with usual manners by introducing a substituent R¹ into the hydroxyl group at position 4 of the piperidine ring of the compound [I-a] or the compound [I-b] obtained in the manner described above. This reaction can be carried out according to a known method such as that described in International Publication No. WO 2003/099787, and this reaction can be carried out, for example, in the presence or absence of a suitable solvent. The solvent may be any solvent which does not have a negative impact on the reaction, and examples thereof include, for example, esters such as isopropyl acetate and ethyl acetate, aromatic hydrocarbons such as benzene and toluene, ethers such as tert-butyl methyl ether and isopropyl ether or a combination thereof. Among them, a combination of isopropyl acetate and toluene is preferably used. The reaction temperature of this reaction is preferably 10 to 50°C and more preferably 10 to 30°C. The reaction time may vary according to the reaction temperature and the like, and is normally 30 minutes to 24 hours and preferably 1 to 20 hours.

The reaction of the compound [I-c] or the compound [I-d], the compound [IV] and the ureation agent can be carried out by a known method such as that described in International Publication No. WO 2003/099787, and this method can be carried out, for example, in a suitable solvent. Examples of ureation agents include compounds represented by the formula: 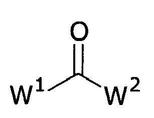 wherein, W¹ and W² may be the same or different and each represents a leaving group. Examples of W¹ and W² may be the same or different and include an imidazolyl group, a halogen atom or a phenoxy group. Specific examples of ureation agents preferably include carbonyldihalides such as 1,1'-carbonyldiimidazole, triphosgene and phosgene. In addition, the solvent employed may be any solvent which does not have a negative impact on the reaction, and examples thereof that can be suitably used include nitriles such as acetonitrile, halogenated hydrocarbons such as dichloromethane and chloroform, and ethers such as diethyl ether and tetrahydrofuran. This reaction can be carried out at, for example, 0 to 80°C and preferably 0 to 60°C. The reaction time may vary according to the reaction temperature and the like, and is normally 30 minutes to 24 hours and preferably 1 to 20 hours.

The reaction of the compound [1-c] or the compound [1-d], the compound [IV] and the ureation agent can also be carried out in, for example, the manner indicated below. A compound [V-a] or a compound [V-b] can be prepared by:
reacting the compound [I-c] or the compound [I-d] with an ureation agent represented by the formula: 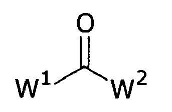
wherein, W¹ and W² have the same meaning as defined above, to obtain a compound represented by general formula [VI-a] or general formula [VI-b]: 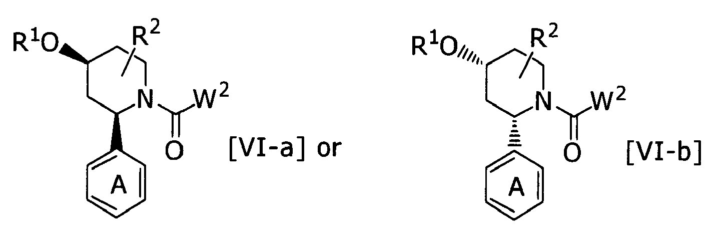 wherein, ring A, R¹, R² and W² have the same meaning as defined above, followed by
as desired, deriving the compound [VI-a] or the compound [VI-b] to a reactive derivative thereof, and
reacting with the compound [IV].
Alternatively, the compound [V-a] or the compound [V-b] can be prepared by:
reacting the compound [IV] with an ureation agent represented by the formula: 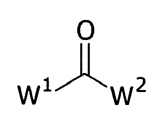
wherein, W¹ and W² have the same meaning as defined above, to obtain a compound represented by general formula [VII]: 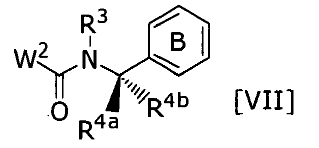 wherein, ring B, R³, R^{4a}, R^{4b} and W² have the same meaning as defined above, followed by
as desired, deriving the compound [VII] to a reactive derivative thereof, and
reacting with the compound [I-c] or the compound [I-d].

Examples of reactive derivatives of the compound [VIa] or the compound [VI-b], or the compound [VII] include those compounds in which W² has been derived to a group as represented by the formula: 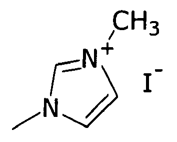 in the compound [VI-a] or the compound [VI-b], or the compound [VII].

The reaction of the compound [I-c] or the compound [I-d], or the compound [IV] with the ureation agent can be carried out in a suitable solvent. The reaction temperature of this reaction is, for example, 0 to 80°C and preferably 0 to 60°C. The reaction time may vary according to the reaction temperature and the like, and is normally 30 minutes to 24 hours and preferably 1 to 20 hours. In addition, the solvent employed may be any solvent which does not have a negative impact on the reaction, and examples of solvents that can be used suitably include nitriles such as acetonitrile, halogenated hydrocarbons such as dichloromethane and chloroform, and ethers such as diethyl ether and tetrahydrofuran.

The reaction for deriving the compound [VI-a] or the compound [VI-b], or the compound [VII] to a reactive derivative thereof can be carried out in a solvent using a reactive derivatizing agent such as methyl iodide. The reaction temperature of this reaction is, for example, 0 to 80°C and preferably 0 to 60°C. The reaction time may vary according to the reaction temperature and the like, and is normally 30 minutes to 24 hours and preferably 1 to 20 hours. In addition, the solvent employed may be any solvent which does not have a negative impact on the reaction, and examples of solvents that can be used suitably include nitriles such as acetonitrile, halogenated hydrocarbons such as dichloromethane and chloroform, and ethers such as diethyl ether and tetrahydrofuran.

The reaction of each reactive derivative with the compound [I-a] or the compound [I-b], or the compound [IV] can be carried out in a suitable solvent in the presence of a base. The reaction temperature of this reaction is, for example, 0 to 80°C and preferably 0 to 60°C. The reaction time may vary according to the reaction temperature and the like, and is normally 30 minutes to 24 hours and preferably 1 to 20 hours. In addition, an amine such as triethylamine can be used for the base. In addition, the solvent employed may be any solvent which does not have a negative impact on the reaction, and examples of solvents that can be used suitably include nitriles such as acetonitrile, halogenated hydrocarbons such as dichloromethane and chloroform, and ethers such as diethyl ether and tetrahydrofuran.

The ring A in the compound used or obtained in the present invention is an optionally substituted benzene ring. Examples of substituents of the benzene ring include an alkyl group, a halogen atom, a cyano group, an optionally protected hydroxyl group and an alkoxy group. The ring A may have 1 to 3 substituents as mentioned above, which may be the same or different.

The ring B in the compound used or obtained in the present invention is an optionally substituted benzene ring. Examples of substituents of the benzene ring include a trihalogenoalkyl group, a halogen atom, a cyano group, a phenyl group, a heterocyclic group containing as a heteroatom 1 to 4 atoms selected from nitrogen atom, oxygen atom and sulfur atom, an alkyl group, an optionally protected hydroxyl group and an alkoxy group. The ring B may have 1 to 3 substituents as mentioned above, which may be the same or different.

Preferred examples of the ring A in the compound used or obtained in the present invention include, for example, benzene rings represented by the formula: 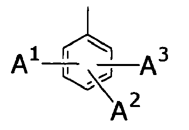 wherein, A¹, A² and A³ may be the same or different and each represents a hydrogen atom, a halogen atom, an alkyl group, an optionally protected hydroxyl group or an alkoxy group.

In addition, preferred examples of the ring B in the compound used or obtained in the present invention include, for example, benzene rings represented by the formula: 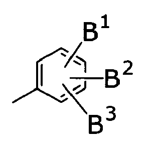 wherein, B¹, B² and B³ may be the same or different and each represents a hydrogen atom, a trihalogenoalkyl group, a halogen atom, a cyano group, a phenyl group, a heterocyclic group containing as a heteroatom 1 to 4 atoms selected from nitrogen atom, oxygen atom and sulfur atom, an alkyl group, an optionally protected hydroxyl group or an alkoxy group.

Examples of the trihalogenoalkyl group include a trifluoromethyl group, trichloromethyl group, etc. Examples of the heterocyclic group containing as a heteroatom 1 to 4 atoms selected from nitrogen atom, oxygen atom and sulfur atom include a tetrazolyl group.

Examples of the protecting groups for the optionally protected hydroxyl group in the compound used or obtained in the present invention include commonly used protecting groups such as an optionally substituted arylalkyl group, an optionally substituted silyl group and an acyl group. Preferred examples thereof include arylalkyl groups such as a benzyl group and phenethyl group, substituted silyl groups such as a tert-butyldimethylsilyl group and tert-butyldiphenylsilyl group, and acyl groups such as a formyl group, acetyl group, propionyl group, malonyl group, acryloyl group and benzoyl group.

Examples of R¹ in the compound used or obtained in the present invention include:
(1) a hydrogen atom,
(2) a substituted carbonyl group,
(3) a substituted sulfinyl group,
(4) a substituted sulfonyl group, or
(5) an optionally substituted alkyl group.
Among them, a substituted carbonyl group and an optionally substituted alkyl group are preferred.

Examples of substituents of the (2) substituted carbonyl group include an optionally substituted alkyl group, optionally substituted alkoxy group, substituted amino group, and monocyclic heterocyclic group containing as a heteroatom 1 to 2 atoms selected from nitrogen atom and oxygen atom (and the monocyclic heterocyclic group is optionally substituted). Examples of substituents of the optionally substituted alkyl group include a hydroxyl group. Examples of substituents of the optionally substituted alkoxy group include an alkoxy group, hydroxyl group and halogen atom. Examples of substituents of the substituted amino group include: an alkyl group substituted with a group selected from a halogen atom, dialkylamino group, piperidinyl group, morpholino group, carboxyl group, morpholinocarbonyl group, dialkylaminocarbonyl group, alkylaminocarbonyl group, alkanoylamino group, alkylthio group, alkoxy group, alkylsulfonyl group, alkanoyloxy group and hydroxyl group; a piperidinyl group substituted with a hydroxyalkanoyl group or alkoxyalkanoyl group; or a dialkylaminosulfonyl group. Examples of the monocyclic heterocyclic group include a morpholino group, piperazinyl group, imidazolyl group, thiomorpholino group, piperidino group, furyl group, tetrahydrothiazolinyl group or pyrrolidinyl group. Examples of substituents of the monocyclic heterocyclic group include a hydroxyl group, alkoxycarbonyl group, carboxyl group, hydroxyalkylaminocarbonyl group, alkoxyalkylaminocarbonyl group, alkylthioalkylaminocarbonyl group, alkylsulfinylalkylaminocarbonyl group, alkylsulfonylalkylaminocarbonyl group, or an alkyl group, oxo group or hydroxyl group optionally substituted with a morpholino group.

Examples of substituents of the (3) substituted sulfinyl group include an alkyl group or a thienyl group.

Examples of substituents of the (4) substituted sulfonyl group include an alkyl group or a thienyl group.

Examples of substituents of the (5) optionally substituted alkyl group include an optionally substituted hydroxyl group, dialkylamino group or monocyclic heterocyclic group containing as a heteroatom 1 to 4 atoms selected from sulfur atom, nitrogen atom and oxygen atom (and the monocyclic heterocyclic group is optionally substituted). Examples of substituents of the optionally substituted hydroxyl group include an alkyl group, alkylsulfonyl group or tetrahydropyranyl group. Examples of the monocyclic heterocyclic group include a pyridyl group, piperidinyl group, morpholino group, isoxazolyl group, triazolyl group, tetrazolyl group or pyrrolidinyl group. Examples of substituents of the monocyclic heterocyclic group include an alkyl group and a phenyl group.

Preferred examples of R¹ include a hydrogen atom; an alkyl group substituted with a hydroxyl group, alkylsulfonyloxy group, tetrahydropyranyloxy group, triazolyl group, tetrazolyl group optionally substituted with an alkyl group or alkoxy group; a morpholinocarbonyloxy group; an imidazolylcarbonyl group; an alkylaminocarbonyl group in which the alkyl group moiety is optionally substituted with a hydroxyl group, morpholinocarbonyl group, dialkylaminocarbonyl group, alkylaminocarbonyl group, alkanoylamino group, alkoxy group or alkanoyloxy group; a piperidinocarbonyl group substituted with a hydroxyl group, carboxyl group, hydroxyalkylaminocarbonyl group, alkoxyalkylaminocarbonyl group, alkylthioalkylaminocarbonyl group or hydroxyalkyl group; a dialkylaminocarbonyl group substituted with a hydroxyl group; a piperidinylaminocarbonyl group substituted with a hydroxyalkanoyl group or alkoxyalkanoyl group; an oxopyrrolidinylcarbonyl group; and a dialkylaminosulfonylaminocarbonyl group.

R² in the compound used or obtained in the present invention represents a hydrogen atom, an optionally substituted hydroxyl group, an optionally substituted amino group, an optionally substituted alkyl group, a substituted carbonyl group or a halogen atom. An example of a substituent of the optionally substituted hydroxyl group of R² is an alkyl group. An example of a substituent of the optionally substituted amino group of R² is an alkyl group. An example of a substituent of the optionally substituted alkyl group of R² is an alkoxy group. Examples of a substituent of the substituted carbonyl group of R² include a hydroxyl group, alkoxy group or alkylamino group. A preferred example of R² is a hydrogen atom.

Examples of R³ in the compound used or obtained in the present invention include a hydrogen atom or an optionally substituted alkyl group. Examples of a substituent of the optionally substituted alkyl group of R³ include a hydroxyl group, alkanoyl group, alkanoyloxy group, halogen atom, alkoxy group or alkylamino group. Preferred examples of R³ include an alkyl group, an alkyl group substituted with a hydroxyl group, or an alkyl group substituted with an alkanoyloxy group. More preferred examples of R³ include a methyl group, ethyl group, propyl group substituted with a hydroxyl group or propyl group substituted with an acetyloxy group.

R^{4a} and R^{4b} in the compound used or obtained in the present invention may be the same or different and each represents a hydrogen atom or an optionally substituted alkyl group, or R^{4a} and R^{4b} taken together form an alkylene group. An example of a substituent of the optionally substituted alkyl group is a hydroxyl group, etc. Preferred examples of R^{4a} and R^{4b} include a hydrogen atom, methyl group or methyl group substituted with a hydroxyl group.

An example of R in the compound used or obtained in the present invention is a protecting group for an amino group. Examples of the protecting group for an amino group include an alkoxycarbonyl group such as a tert-butoxycarbonyl group, an arylalkoxycarbonyl group such as a benzyloxycarbonyl group, and the like. A preferred example of R is a tert-butoxycarbonyl group.

The compound used or obtained in the present invention is preferably those compounds in which ring A is a benzene ring represented by the formula: 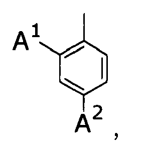 A¹ is a hydrogen atom, alkyl group or halogen atom and A² is a hydrogen atom, alkyl group or halogen atom, more preferably those compounds in which A¹ is an alkyl group and A² is a halogen atom, and further more preferably those compounds in which A¹ is a methyl group and A² is a fluorine atom.

The compound used or obtained in the present invention is preferably those compounds in which ring B is a benzene ring represented by the formula: 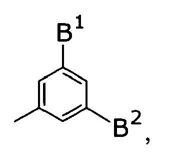 B¹ is a trihalogenoalkyl group and B² is a trihalogenoalkyl group, and more preferably those compounds in which B¹ is a trichloromethyl group and B² is a trichloromethyl group.

In addition, the compound used or obtained in the present invention is preferably those compounds in which R¹ is a hydrogen atom or hydroxyalkylaminocarbonyl group, R² is a hydrogen atom, R³ is an alkyl group, and R^{4a} and R^{4b} may be the same or different and each is a hydrogen atom or alkyl group.

The compound used or obtained in the present invention can be used in a free form, or in the form of a salt or a pharmaceutically acceptable salt.

Examples of salts or pharmaceutically acceptable salts of the compounds used or obtained in the present invention include inorganic acid salts such as hydrochlorides, sulfates, phosphates and hydrobromides, and organic acid salts such as acetates, fumarates, oxalate, citrate, methanesulfonate, benzenesulfonates, tosylates, maleates, succinates and tartrates.

In addition, the compound used or obtained in the present invention, or a salt thereof or a pharmaceutically acceptable salt thereof include any of intramolecular salts, and solvates or hydrates thereof.

As described in International Publication No. WO 2003/099787, the compound [V-a] or compound [V-b] obtained in the present invention, or a pharmaceutically acceptable salt thereof, has excellent tachykinin receptor antagonistic action, in particular, SP receptor antagonistic action, and therefore is useful as safe prophylactic or therapeutic agents for inflammation or allergic diseases (for example, atopic dermatitis, dermatitis, herpes, psoriasis, asthma, bronchitis, sputum, rhinitis, rheumatoid arthritis, osteoarthritis, osteoporosis, multiple sclerosis, conjunctivitis, ophthalmia, cystitis, etc), pain, migraine, neuralgia, itching and coughing, as well as diseases of the central nervous system [for example, schizophrenia, Parkinson's disease, depression, anxiety, psychosomatic disorders, morphine dependency, dementia (for example, Alzheimer's disease, etc), etc], digestive tract diseases [for example, irritable bowel syndrome, ulcerative colitis, Crohn's disease, abnormalities caused by urease-positive, helical gram-negative bacteria (e.g., *Helicobacter pylori*, etc) (for example, gastritis, gastric ulcer, etc), etc], nausea, vomiting, urinary disorders (for example, pollakiuria, urinary incontinence, etc), circulatory diseases (for example, angina pectoris, hypertension, cardiac insufficiency, thrombosis, etc), and immune diseases in mammals (for example, mouse, guinea pig, gerbil, ferret, rat, hamster, rabbit, cat, dog, cow, sheep, monkey, human, etc). In particular, the compound [V-a] or the compound [V-b] of the present invention, or a pharmaceutically acceptable salt thereof, exhibits high intracerebral migration, low toxicity and hardly any adverse side effects. Therefore, it is useful as a prophylactic or therapeutic agent for vomiting, diseases of the central nervous system such as depression, and urinary disorders such as pollakiuria.

Moreover, during preparation of the compounds according to the present invention, in cases in which the starting compounds and/or the respective intermediate compounds have functional groups, suitable protecting groups other than those described hereinbefore may be introduced for each functional group in accordance with usual manners of synthetic chemistry, and those protecting groups may also be suitably removed as necessary.

As used herein, alkyl groups refer to, for example, linear or branched alkyl groups having 1 to 6 carbon atoms, such as a methyl group, ethyl group, propyl group, isopropyl group, butyl group, isobutyl group, tert-butyl group, pentyl group, isopentyl group and hexyl group, preferably refer to those having 1 to 4 carbon atoms, and more preferably refer to those having 1 to 2 carbon atoms. Alkoxy groups refer to, for example, linear or branched alkoxy groups having 1 to 6 carbon atoms, such as a methoxy group, ethoxy group, propoxy group, isopropoxy group and butoxy group, preferably refer to those having 1 to 4 carbon atoms, and more preferably refer to those having 1 to 2 carbon atoms. Alkanoyl groups refer to, for example, linear or branched alkanoyl groups having 1 to 6 carbon atoms, such as a formyl group, acetyl group, propionyl group, butyryl group, valeryl group and tert-butylcarbonyl group, preferably refer to those having 1 to 4 carbon atoms, and more preferably refer to those having 1 to 2 carbon atoms. Alkylene groups refer to, for example, alkylene groups having 1 to 6 carbon atoms such as a methylene group, ethylene group, propylene group, trimethylene group, tetramethylene group, pentamethylene group and hexamethylene group, and preferably refer to those having 2 to 5 carbon atoms. Moreover, halogen atoms refer to chlorine, bromine, fluorine or iodine, and preferably include chlorine or fluorine.

### EXAMPLES

### Example 1

To a solution of 288 mg of sodium hydroxide added in 20 ml of methanol was added 1.82 g of 1-tert-butoxycarbonyl-2-phenyl-2,3-dihydro-1H-4-oxo-pyridine at 30°C or lower. The reaction solution was heated to 50°C and stirred for 2 hours at the same temperature to give 2,3-dihydro-4-sodiumoxy-2-phenylpyridine. The reaction solution was then cooled to 25°C, and 4 ml of isopropyl alcohol was added followed by 496 mg of sodium borohydride. The reaction solution was then stirred for 16 hours at 35°C. Subsequently, to the reaction solution was added 6 g of 20% aqueous sodium hydroxide solution at 30°C or lower, and the reaction solution was stirred for 1 hour at 25°C. After concentrating the reaction solution, 10 ml of water was added to the residue and the mixture was stirred for 1 hour at 50°C. After allowing the solution to cool to 30°C or lower, 16 ml of isopropyl acetate was added to separate the solution. The aqueous layer was extracted again with isopropyl acetate. The combined organic layers were washed with 20% brine and water. The resulting organic layer was then concentrated under reduced pressure to give 1.18 g of 4-hydroxy-2-phenylpyridine shown in the following Table 1 (ratio of syn/anti forms = 9.8/1.0).
MS: 178 (M+1).

### Method for Determining Syn/Anti Ratio:

The syn/anti ratio was determined from the NMR peak ratio.

### <1H-NMR Measurement>

Measurement of 1H-NMR (Nuclear Magnetic Resonance) was carried out in CDCl₃ using BRUKER AVANCE 400.

### Examples 2 to 7

Compounds shown in the following Table 1 were obtained by using the corresponding starting compounds and treating in the same manner as Example 1.

### Example 8

To a solution of 39 mg of sodium hydroxide added in 60 ml of methanol was added 6.00 g of 1-tert-butoxycarbonyl-2-(4-fluoro-2-methylphenyl)-2,3-dihydro-1H-4-oxo-pyridine at 30°C or lower. The reaction solution was heated to 40°C and stirred for 3 hours at the same temperature to give 2-(4-fluoro-2-methylphenyl)-2,3-dihydro-4-sodiumoxy-pyridine. After distilling off the solvent, replacing with ethanol and concentrating, to 4.12 g of the resulting residue were added 36 ml of ethanol and 36 ml of isopropanol, and the mixture was heated to 50°C. Subsequently, 0.96 g of sodium borohydride was added and the reaction solution was stirred for 2 hours at the same temperature. Subsequently, to the reaction solution was added 17 ml of 10% aqueous sodium hydroxide solution at 30°C or lower, and the mixture was stirred for 1 hour. After concentrating the reaction solution, water and ethyl acetate were added to the residue to separate the solution. The aqueous layer was extracted again with ethyl acetate. The combined organic layers were washed with 20% brine and water. The resulting organic layer was concentrated under reduced pressure to give 4.18 g of 2-(4-fluoro-2-methylphenyl)-4-hydroxypiperidine (ratio of syn/anti forms = 13/1). MS: 210 (M+1).

### Example 9

(1) To 12.4 g of 2-(4-fluoro-2-methylphenyl)-4-hydroxypiperidine was added 50 ml of methanol, and the mixture was heated to 60°C. To the solution was added dropwise 25 ml of a solution of 3.79 g of N-tosyl-D-phenylalanine in methanol, and the mixture was stirred for 1 hour at 60°C. Moreover, 62 ml of a solution of 8.52 g of N-tosyl-D-phenylalanine in methanol was added dropwise at 60°C over 1 hour. After allowing the reaction solution to cool to 15°C, the reaction solution was stirred for 1 hour. The precipitated crystals were collected by filtration, washed with cold methanol, and then dried under reduced pressure at 50°C to give 12.48 g of (2R,4S)-2-(4-fluoro-2-methylphenyl)-4-hydroxypiperidine·N-tosyl-D-phenylalanine salt having an optical purity of 99.9% ee (the optical purity was measured by HPLC in accordance with usual manners).
(2) 36.0 g of the resulting (2R,4S)-2-(4-fluoro-2-phenylmethyl)-4-hydroxypiperidine·N-tosyl-D-phenylalanine salt was added to a mixed solution of 144 ml of isopropyl acetate, 93.6 ml of water and 14.4 g of concentrated hydrochloric acid at 40°C, and the mixture was stirred for 30 minutes. The reaction solution was separated and the separated organic layer was collected. The remaining aqueous layer was again extracted with isopropyl acetate and the organic layer was collected by separation. Moreover, after adding 7 M aqueous sodium hydroxide solution to the remaining aqueous layer at 45°C or lower to adjust the pH to about 11, the resulting solution was extracted twice with isopropyl acetate and the organic layer was collected by separation. The resulting organic layers were combined and the combined organic layers were concentrated to one-fifth of its original volume under reduced pressure. After adding 180 ml of isopropyl acetate to the residue and dissolving the residue therein, 3.6 g of toluene was added. To the solution was added 15.35 g of 1,1'-carbonyldiimidazole in nitrogen atmosphere, and the mixture was stirred for 30 minutes at 15°C. The reaction solution was then washed twice with water. The resulting organic layer was heated to 50°C and 6.2 g of ethanolamine was added thereto, and the mixture was stirred for 3 hours at the same temperature. Then, 36 ml of water was added to the solution, and the mixture was cooled to 10°C and stirred for 1 hour. The precipitated crystals were collected by filtration and washed with water and isopropyl acetate cooled to 10°C. The resulting crystals were then dried with blowing air at 45°C to give 17.71 g of (2R,4S)-2-(4-fluoro-2-methylphenyl)-4-(2-hydroxyethylaminocarbonyloxy)piperidine·monohydrate. MS: 297 (M+1).

### Example 10

A mixed solvent of 11 ml of isopropyl acetate and 5.5 ml of water was heated to 60°C, and thereto were added 1.15 g of 4-hydroxy-2-phenylpiperidine obtained in Example 1 followed by 1 g of potassium carbonate. Moreover, 2.2 g of N-(3,5-bistrifluoromethylbenzyl)-N-methyl-aminocarbonyl chloride was added to the solution at 60°C, and the mixture was stirred for 19 hours. After cooling to 40°C, 11 ml of toluene was added to the reaction solution, and the solution was washed with aqueous hydrochloric acid solution. The organic layer was concentrated under reduced pressure, and the residue was purified by silica gel chromatography (hexane:ethyl acetate = 80:20 → 0:100) to give 2.48 g of 1-{N-(3,5-bistrifluoromethylbenzyl)-N-methyl}aminocarbonyl-2-phenyl-4-hydroxypiperidine. MS: 461 (M+1).

### Example 11

The treatment was carried out in the same manner as Example 10 using 12.72 g of (2R,4S)-2-(4-fluoro-2-methylphenyl)-4-(2-hydroxyethylaminocarbonyloxy)piperidine·monohydrate and 13.58 g of N-(3,5-bistrifluoromethylbenzyl)-N-methylaminocarbonyl chloride to give 8.82 g of (2R,4S)-1-{N-(3,5-bistrifluoromethylbenzyl)-N-methyl}aminocarbonyl-2-(4-fluoro-2-methylphenyl)-4-(2-hydroxyethylaminocarbonyloxy)piperidine. MS: 580 (M+1).

### Example 12

The treatment was carried out in the same manner as Example 10 using 1.35 g of 2-(2,4-dimethylphenyl)-4-hydroxypiperidine obtained in Example 4 and 2.2 g of N-(3,5-bistrifluoromethylbenzyl)-N-methyl-aminocarbonyl chloride to give 2.79 g of 1-{N-(3,5-bistrifluoromethylbenzyl)-N-methyl}aminocarbonyl-2-(2,4-dimethylphenyl)-4-hydroxypiperidine. MS: 489 (M+1).

### Examples 13 to 85

The compounds shown in the following Tables 2 to 14 can be obtained by treating the corresponding starting compounds according to the methods of Example 1, Example 9 and Example 10.

### Reference Example 1

To 45 ml of tetrahydrofuran were added 9.09 g of 4-methoxypyridine and 19.93 g of di-tert-butylcarbonate in nitrogen atmosphere, and the solution was cooled to -10°C. To the solution was added dropwise 100 ml of a solution of 1M phenylmagnesium bromide in tetrahydrofuran at -10°C over 2 hours, and the mixture was stirred for 3 hours at - 10°C. The reaction solution was then added to a solution of 19.09 g of citric acid dissolved in 71.8 ml of water at 30°C or lower, and the mixture was stirred for 45 minutes at 30°C or lower. After separating the solution, the organic layer was washed with brine. The organic layer was concentrated and the residue was purified by silica gel chromatography (hexane:ethyl acetate = 100:0 → 60:40) to give 19.66 g of 1-tert-butoxycarbonyl-2-phenyl-2,3-dihydro-1H-4-oxopyridine shown in the following Table 15.

### Reference Examples 2 to 7

The compounds shown in the following Table 15 were obtained by using the corresponding starting compounds and treating in the same manner as Reference Example 1.

**Table 1**

| | | | |
|---|---|---|---|
| 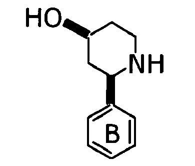 | | | |

| Example No. | Ring B | syn/anti ratio | MS |
|---|---|---|---|
| 1 | 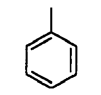 | 9.8/1.0 | 178(M+1) |
| 2 | 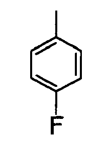 | 9.6/1.0 | 196(M+1) |
| 3 | 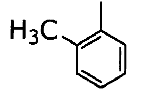 | 13.1/1.0 | 192(M+1) |
| 4 | 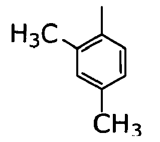 | 12.4/1.0 | 206(M+1) |
| 5 | 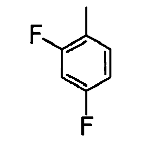 | 10.6/1.0 | 214(M+1) |
| 6 | 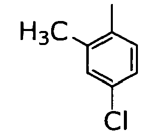 | 19.0/1.0 | 226(M+1) |
| 7 | 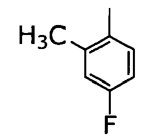 | 13.2/1.0 | 210(M+1) |

**Table 2**

| | |
|---|---|
| 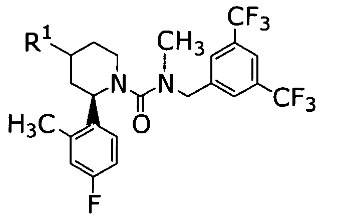 | |

| Example No. | R¹ |
|---|---|
| 13 | 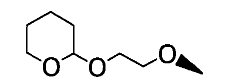 |
| 14 | 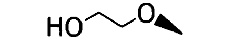 |
| 15 | 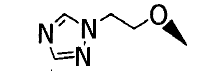 |
| 16 | 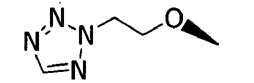 |
| 17 | 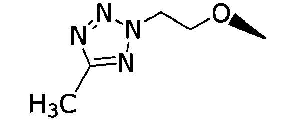 |
| 18 | 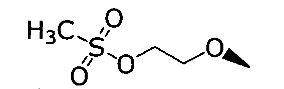 |
| 19 | 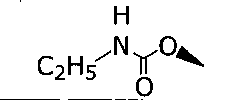 |
| 20 | 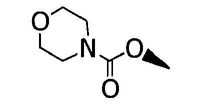 |
| 21 | 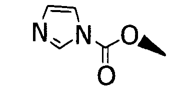 |
| 22 | 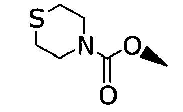 |

**Table 3**

| | |
|---|---|
| 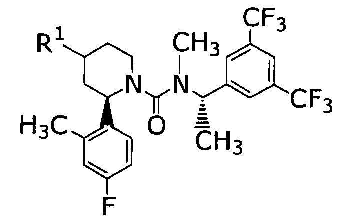 | |

| Example No. | R¹ |
|---|---|
| 23 | 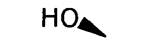 |
| 24 | 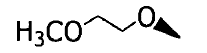 |
| 25 | 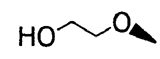 |
| 26 | 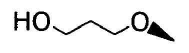 |
| 27 | 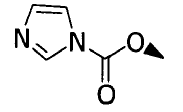 |
| 28 | 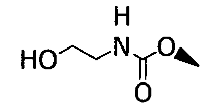 |
| 29 | 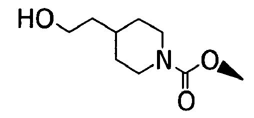 |
| 30 | 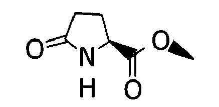 |
| 31 | 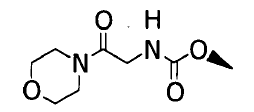 |
| 32 | 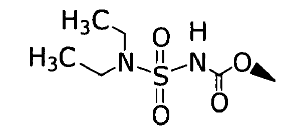 |

**Table 4**

| | |
|---|---|
| 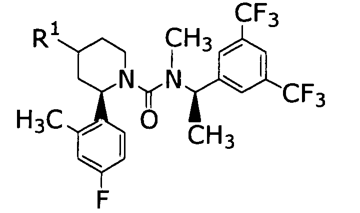 | |

| Example No. | R¹ |
|---|---|
| 33 | 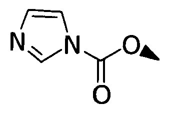 |
| 34 | 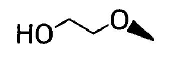 |
| 35 | 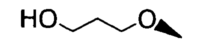 |
| 36 | 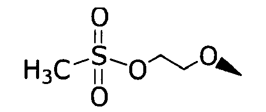 |
| 37 | 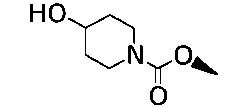 |
| 38 | 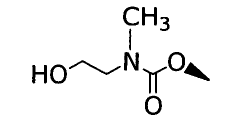 |
| 39 | 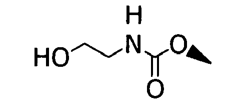 |

**Table 5**

| | |
|---|---|
| 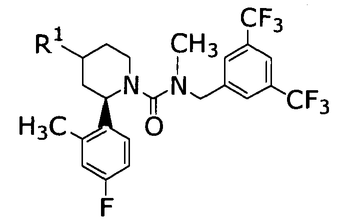 | |

| Example No. | R¹ |
|---|---|
| 40 | 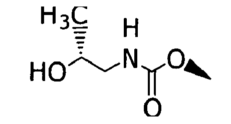 |
| 41 | 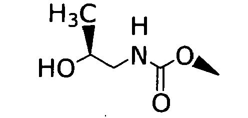 |
| 42 | 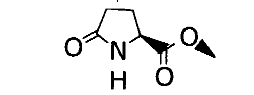 |
| 43 | 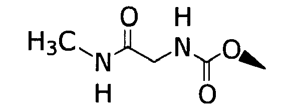 |
| 44 | 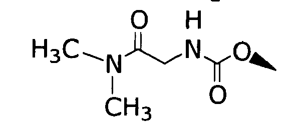 |
| 45 | 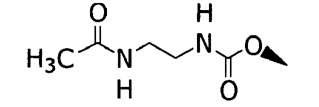 |
| 46 | 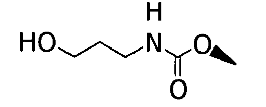 |

**Table 6**

| | |
|---|---|
| 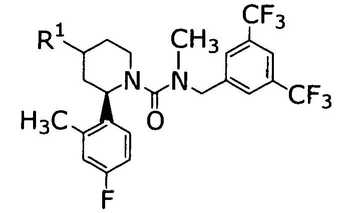 | |

| Example No. | R¹ |
|---|---|
| 47 | 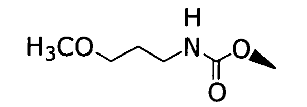 |
| 48 | 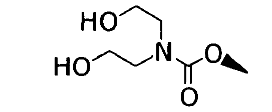 |
| 49 | 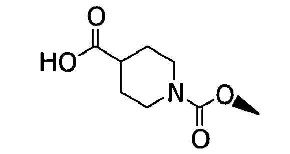 |
| 50 | 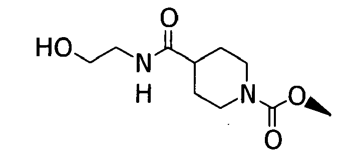 |
| 51 | 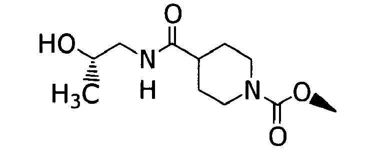 |
| 52 | 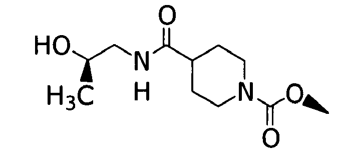 |
| 53 | 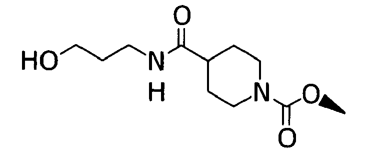 |

**Table 7**

| Example No. | Structural formula |
|---|---|
| 54 | 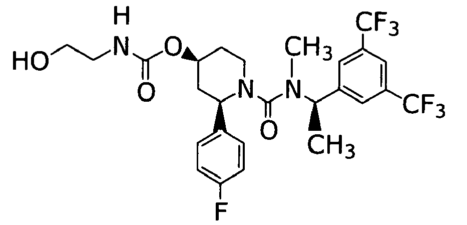 |
| 55 | 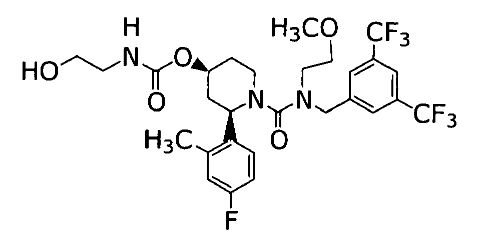 |
| 56 | 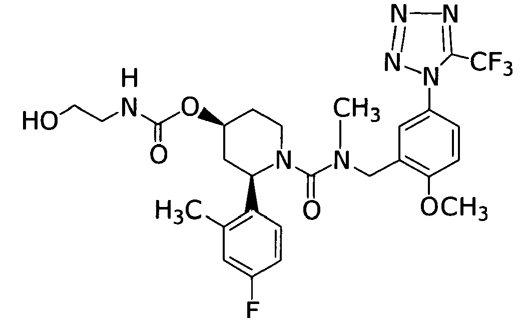 |

**Table 8**

| | |
|---|---|
| 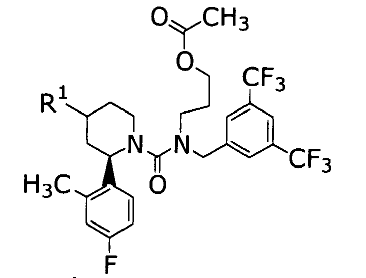 | |

| Example No. | R¹ |
|---|---|
| 57 | 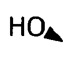 |
| 58 | 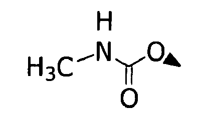 |
| 59 | 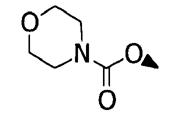 |
| 60 | 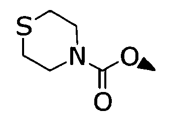 |
| 61 | 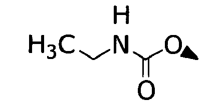 |

**Table 9**

| | |
|---|---|
| 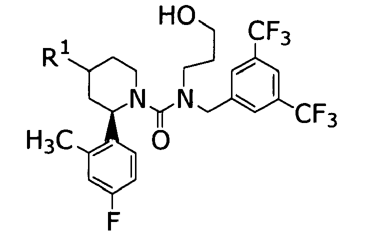 | |

| Example No. | R¹ |
|---|---|
| 62 | 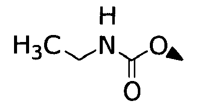 |
| 63 | 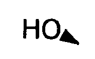 |
| 64 | 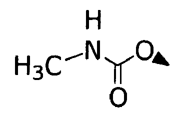 |
| 65 | 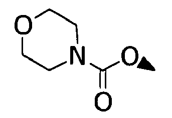 |
| 66 | 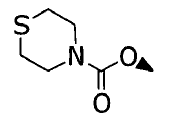 |

**Table 10**

| | |
|---|---|
| 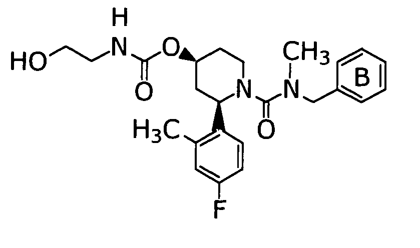 | |

| Example No. | Ring B |
|---|---|
| 67 | 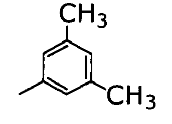 |
| 68 | 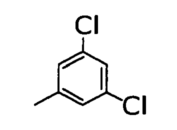 |

**Table 11**

| Example No. | Structural formula |
|---|---|
| 69 | 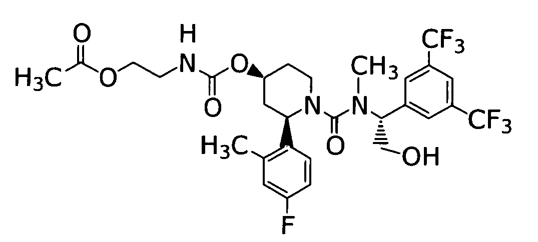 |
| 70 | 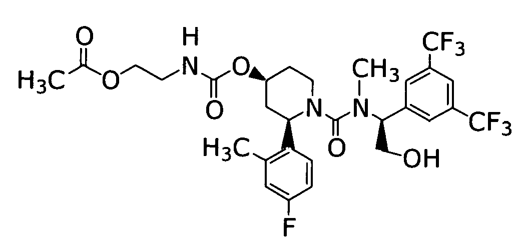 |
| 71 | 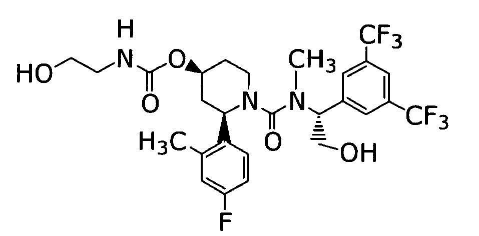 |
| 72 | 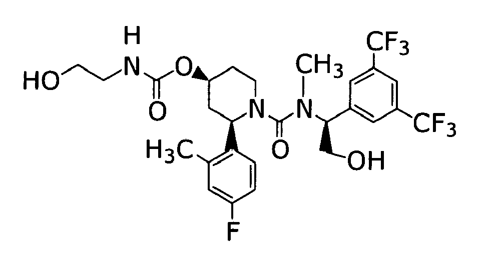 |

**Table 12**

| | |
|---|---|
| 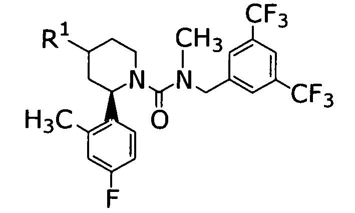 | |

| Example No. | R¹ |
|---|---|
| 73 | 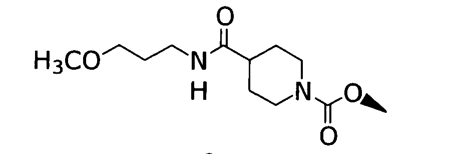 |
| 74 | 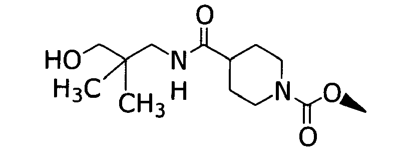 |
| 75 | 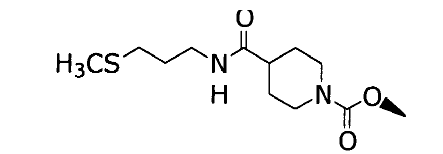 |
| 76 | 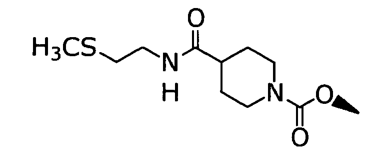 |
| 77 | 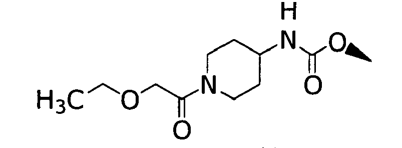 |
| 78 | 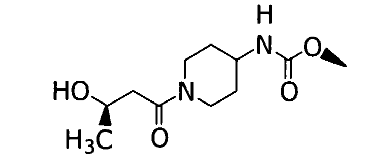 |

**Table 13**

| | |
|---|---|
| | 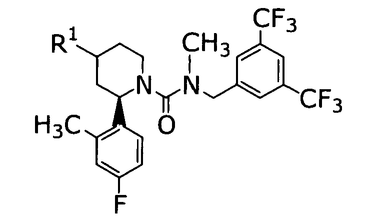 |

| Example No. | R¹ |
|---|---|
| 79 | 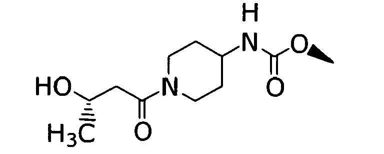 |
| 80 | 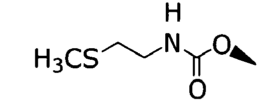 |
| 81 | 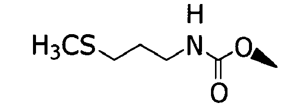 |
| 82 | 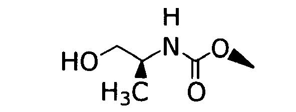 |
| 83 | 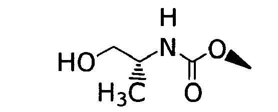 |

**Table 14**

| | |
|---|---|
| | 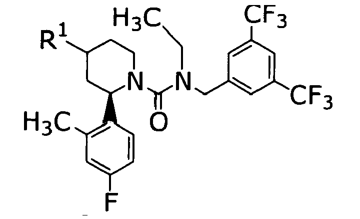 |

| Example No. | R¹ |
|---|---|
| 84 | 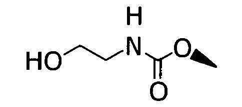 |
| 85 | 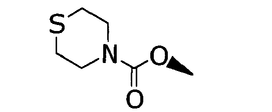 |

**Table 15**

| | | |
|---|---|---|
| 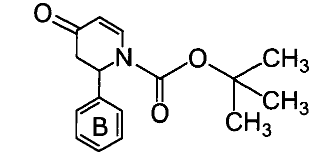 | | |

| Example No. | Ring B | MS |
|---|---|---|
| 1 | 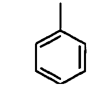 | 274(M+1) |
| 2 | 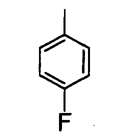 | 292(M+1) |
| 3 | 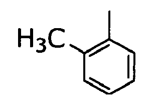 | 288(M+1) |
| 4 | 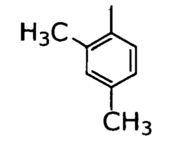 | 302(M+1) |
| 5 | 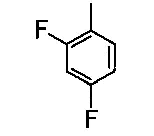 | 310(M+1) |
| 6 | 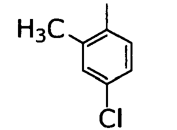 | 322(M+1) |
| 7 | 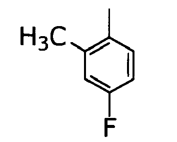 | 306(M+1) |

### INDUSTRIAL APPLICABILITY

According to the preparation methods of the present invention, optically active piperidine compounds having excellent tachykinin receptor antagonistic action, and intermediates thereof: syn-form piperidine compounds and optically active piperidine compounds, can be efficiently produced.

## Claims

1. A method for preparing a syn-form piperidine compound represented by general formula [I]: 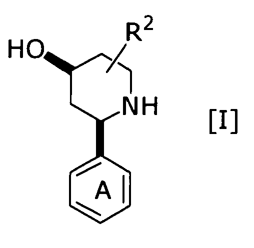 wherein,
bold lines represent bonds in which substituents at positions 2 and 4 of a piperidine ring are in the syn configuration, and the other symbols have the same meaning as defined below, or a salt thereof,
comprising:
reducing a compound represented by general formula [II]: 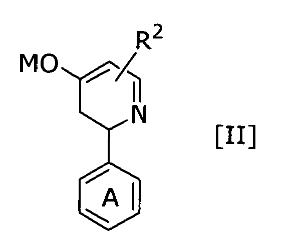
wherein,
ring A represents an optionally substituted benzene ring,
R² represents a hydrogen atom, an optionally substituted hydroxyl group, an optionally substituted amino group, an optionally substituted alkyl group, a substituted carbonyl group or a halogen atom, and
M represents an alkaline metal or a hydrogen atom.

2. A method for preparing a syn-form piperidine compound represented by general formula [I]: 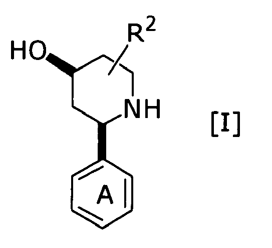 wherein,
bold lines represent bonds in which substituents at positions 2 and 4 of a piperidine ring are in the syn configuration, and the other symbols have the same meaning as defined below, or a salt thereof,
comprising:
reacting a compound represented by general formula [III]: 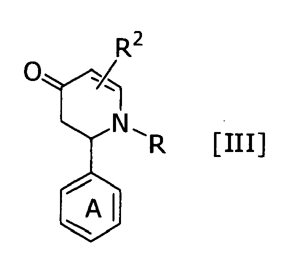
wherein,
ring A represents an optionally substituted benzene ring,
R² represents a hydrogen atom, an optionally substituted hydroxyl group, an optionally substituted amino group, an optionally substituted alkyl group, a substituted carbonyl group or a halogen atom, and
R represents a protecting group for an amino group, or a salt thereof,
with a base to give a compound represented by general formula [II]: 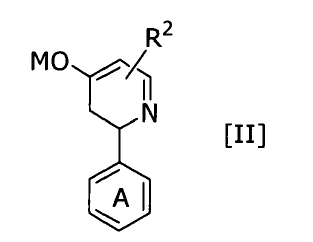 wherein,
M represents an alkaline metal or a hydrogen atom, and the other symbols have the same meaning as defined above, followed by
reducing the compound [II] obtained.

3. The method according to claim 1 or claim 2, wherein the ring A is a benzene ring represented by the formula: 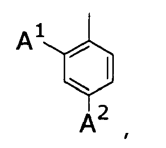 A¹ is a hydrogen atom, an alkyl group or a halogen atom, A² is a hydrogen atom, an alkyl group or a halogen atom, R² is a hydrogen atom, and R is an alkoxycarbonyl group or an arylalkoxycarbonyl group.

4. The method according to claim 3, wherein the base is an alkaline metal alcolate, and M is an alkaline metal.

5. A method for preparing an optically active piperidine compound represented by general formula [I-a] or general formula [I-b]: 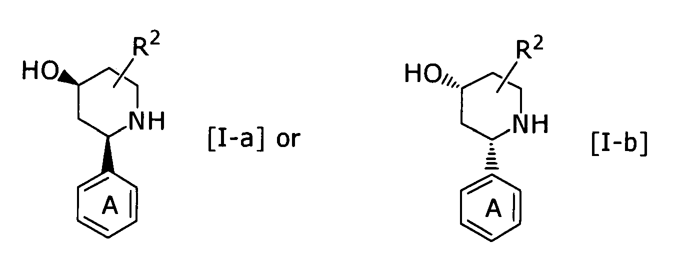 wherein,
the symbols have the same meaning as defined below, comprising:
preparing a syn-form piperidine compound represented by general formula [I]: 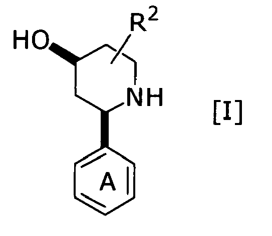
wherein,
ring A represents an optionally substituted benzene ring,
R² represents a hydrogen atom, an optionally substituted hydroxyl group, an optionally substituted amino group, an optionally substituted alkyl group, a substituted carbonyl group or a halogen atom, and
bold lines represent bonds in which substituents at positions 2 and 4 of a piperidine ring are in the syn configuration,
according to the method of claim 1 or claim 2,
allowing the syn-form piperidine compound [I] obtained to act on an optical resolving agent to form two types of diastereomer salts,
using a difference in solubility between the two types of diastereomer salts formed to separate and collect one of the diastereomer salts, and then
decomposing said salt.

6. A method for preparing an optically active piperidine compound represented by general formula [V-a] or general formula [V-b]: 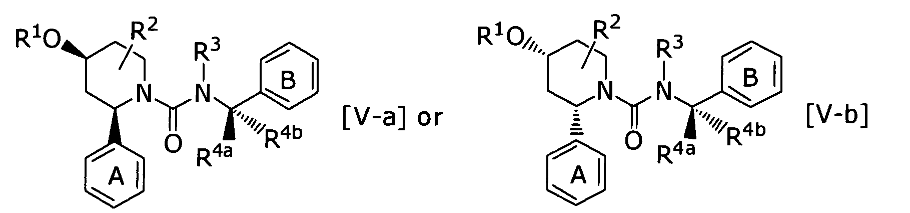 wherein,
the symbols have the same meaning as defined below, or a pharmaceutically acceptable salt thereof,
comprising:
optionally introducing a substituent R¹ to a hydroxyl group at position 4 of a piperidine ring of an optically active piperidine compound represented by general formula [I-a] or general formula [I-b] obtained according to the method of claim 5: 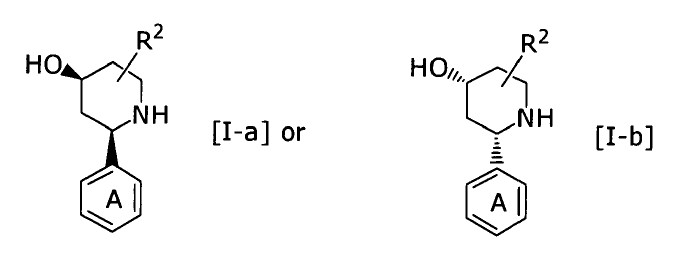
wherein,
ring A represents an optionally substituted benzene ring, and
R² represents a hydrogen atom, an optionally substituted hydroxyl group, an optionally substituted amino group, an optionally substituted alkyl group, a substituted carbonyl group or a halogen atom,
in accordance with usual manners to give a compound represented by general formula [I-c] or general formula [I-d]: 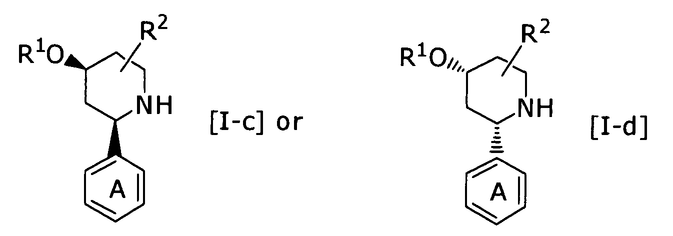 wherein,
R¹ represents a hydrogen atom, a substituted carbonyl group, a substituted sulfinyl group, a substituted sulfonyl group or an optionally substituted alkyl group, and the other symbols have the same meaning as defined above,
reacting the compound of general formula [I-c] or general formula [I-d] obtained, a compound represented by general formula [IV]: 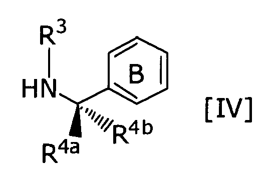 wherein,
ring B represents an optionally substituted benzene ring, R³ represents a hydrogen atom or an optionally substituted alkyl group, R^{4a} and R^{4b} may be the same or different and each represents a hydrogen atom or an optically substituted alkyl group, or R^{4a} and R^{4b} taken together form an alkylene group,
and an ureation agent represented by the formula: 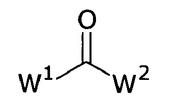 wherein,
W¹ and W² may be the same or different and each represents a leaving group, and then
as desired, converting to a pharmaceutically acceptable salt thereof.
